# EUROPEAN PATENT APPLICATION

(11) **EP 3 192 873 A1**
(43) Date of publication of application: **19.07.2017**
(21) Application number: 17159348.6
(22) Date of filing: 27.09.2010
(51) Int. Cl.: C12N 15/74, C12R 1/225, C12R 1/46, C12N 5/10

(54) **LACTOBACILLUS AND STREPTOCOCCUS PROMOTERS AND USES THEREOF**

(30) Priority: 29.09.2009 EP 09171680
(62) Divisional of application: 10765600.1
(71) Applicant: Intrexon Actobiotics NV, 9052 Zwijnaarde (BE)
(72) Inventor: Steidler, Lothar, 9160 Lokeren (BE); Van Huynegem, Karolien, 9890 Asper (BE)
(74) Representative: Pohlman, Sandra M.

(57) **Abstract**

The invention is in the field of molecular biology, and relates to recombinant engineering and protein expression. More in particular, the invention relates to nucleic acids for recombinant expression of proteins comprising sequences derived from *Lactobacillus* or *Streptococcus* and useful as promoters. The invention further relates to vectors comprising the said nucleic acids and host cells transformed therewith. The invention also covers the use of host cells comprising the said nucleic acids or vectors for expressing heterologous or homologous proteins; and also for delivery, especially therapeutic delivery, of the said proteins to subjects.

## Description

### FIELD OF THE INVENTION

The invention is in the field of molecular biology, and relates to recombinant engineering and protein expression. More in particular, the invention relates to nucleic acids comprising sequences derived from *Lactobacillus* or *Streptococcus* and useful as promoters, to vectors comprising the said nucleic acids and host cells transformed therewith. The invention also covers the use of host cells comprising the said nucleic acids or vectors for expressing heterologous or homologous proteins; and also for delivery, especially therapeutic delivery, of the said proteins to subjects.

### BACKGROUND OF THE INVENTION

Microorganisms such as bacteria, yeast and fungi are increasingly becoming important as hosts for recombinant expression and *in vivo* or *in situ* delivery of prophylactically and/or therapeutically relevant expression products to subjects (e.g., WO 97/14806).

Useful delivery tools may employ *inter alia* bacteria of *Lactobacillus* sp. or *Streptococcus* sp. Many such bacteria are considered as GRAS-microorganisms (*i.e.*, generally regarded as safe) and may be relatively readily administered to humans or animals.

However, achieving strong level of heterologous expression in lactic acid bacteria often requires the introduction of promoters and other sequences that are exogenous to these bacteria and therefore may compromise the GRAS perception thereof.

Accordingly, there exists a need to provide further promoters which are derived from bacteria of *Lactobacillus* sp. or *Streptococcus* sp. and can be favourably used for expression of proteins, preferably heterologous protein expression, in these or related species. Also needed are such promoters which can achieve high expression levels in order to obtain sufficient amounts of so-expressed proteins in industrial and/or therapeutic settings.

### SUMMARY OF THE INVENTION

The aspects of the present invention address at least some, e.g., one or more, of the above discussed needs of the art.

In particular, the present inventors recognised nucleic acids and nucleic acid sequences derived from *Lactobacillus* or *Streptococcus* that can be advantageously used as further promoters for recombinant expression, such as preferably expression of polypeptides, in host cells, preferably in bacteria, more preferably in Gram-positive bacteria, even more preferably in lactic acid bacteria, and most preferably in bacteria of *Lactobacillus* sp. or *Streptococcus* sp.

More in particular, the inventors set out and succeeded to identify nucleic acids and nucleic acid sequences from *Lactobacillus* or *Streptococcus* that can function as strong promoters, i.e., ones that achieve high level of expression, for recombinant expression, such as preferably expression of polypeptides, in host cells, preferably in bacteria, and more preferably in *Lactobacillus* or *Streptococcus.* Strong expression can favourably increase the quantity of expression products, e.g., polypeptides, recombinantly produced by the host cells, that become available for further uses, such as, e.g., for purification from or for therapeutic delivery by the host cells.

The nucleic acids and nucleic acid sequences identified by the inventors are derived from *Lactobacillus* or *Streptococcus,* which may be deemed as GRAS microorganisms. Consequently, compositions, e.g., host cells, comprising such promoters can be administered to humans and animals with less concern for biological safety than when introducing sequences originating from, e.g., non-GRAS microorganisms or other sources.

Thus, the invention provides advantageous *Lactobacillus-derived* or *Streptococcus-derived* nucleic acids and sequences that constitute further promoters, more preferably further strong promoters, for use in numerous applications involving recombinant expression, e.g., of polypeptides, in host cells, preferably in bacteria and even more preferably in bacteria of *Lactobacillus* sp. or *Streptococcus* sp.

The present invention integrates the above relevant realisations in its diverse aspects.

Accordingly, in an aspect, the invention provides a recombinant nucleic acid comprising a promoter, being a native promoter from a *Lactobacillus* species or a functional variant or functional fragment thereof, operably linked to one or more open reading frames heterologous to the promoter, wherein the promoter is chosen from the group comprising or consisting of the native promoters of genes of *Lactobacillus,* preferably but without limitation of *Lactobacillus rhamnosus,* for: 1) ribosomal protein S14 and ribosomal protein S17 and ribosomal protein L15 and ribosomal protein S3 (*rpsJ*), 2) nucleoid DNA-binding protein (*dnabp*), 3) ribosomal protein S21 (*rpS21*), 4) 50S ribosomal protein L19 (*rpIS*), 5) 50S ribosomal protein L17 (*rnap40*), 6) 50S ribosomal protein L13 (*rpIM*), 7) phosphoglycerate mutase 1 (*pgm1*), 8) ribosomal protein S4 (*rpS4*), 9) glyceraldehyde-3-phosphate dehydrogenase (*cggr*), and functional variants and functional fragments of the said native promoters.

In another aspect, the invention provides a recombinant nucleic acid comprising a promoter, being a native promoter from a *Streptococcus* species or a functional variant or functional fragment thereof, operably linked to one or more open reading frames heterologous to the promoter, wherein the promoter is chosen from the group comprising or consisting of the native promoters of genes of *Streptococcus,* preferably but without limitation of *Streptococcus mutans,* for: 10) 30S ribosomal protein S10, 11) 50S ribosomal protein L27, 12) 30S ribosomal protein S15, 13) 30S ribosomal protein S16, 14) 50S ribosomal protein L19, 15) 30S ribosomal protein S8, 16) 50S ribosomal protein L18 and 30S ribosomal protein S5, 17) 30S ribosomal protein S9, 18) 50S ribosomal protein L17, 19) 30S ribosomal protein S13, 20) 30S ribosomal protein S7, 21) 50S ribosomal protein L15, 22) 30S ribosomal protein S4, 23) 50S ribosomal protein L6, 24) 30S ribosomal protein S3 and 50S ribosomal protein L3, 25) phosphoglyceromutase, and functional variants and functional fragments of the said native promoters.

In related exemplary aspects, also provided are vectors comprising the recombinant nucleic acids of the invention; chromosomally integrated expression cassettes, host cells transformed with the recombinant nucleic acids of the invention or with vectors comprising such; the use of the recombinant nucleic acids of the invention for achieving expression of expression products, preferably of one or more polypeptides, encoded by the said open reading frames, in a host cell; methods for recombinant expression and isolation of expression products, preferably polypeptides, of interest using said host cells; treatment methods involving *in situ* delivery of therapeutically relevant expression products, preferably polypeptides, e.g., antigens and/or non-vaccinogenic therapeutically active polypeptides, to humans or animals by such host cells; and related uses of the host cells for the manufacture of medicaments to facilitate the said delivery; pharmaceutical compositions comprising the said host cells; etc.

These and further aspects and preferred embodiments of the invention are described in the following sections and in the appended claims.

### DETAILED DESCRIPTION OF THE INVENTION

As used herein, the singular forms "a", "an", and "the" include both singular and plural referents unless the context clearly dictates otherwise. By way of example, "a cell " refers to one or more than one cell.

The terms "comprising", "comprises" and "comprised of" as used herein are synonymous with "including", "includes" or "containing", "contains", and are inclusive or open-ended and do not exclude additional, non-recited members, elements or method steps. The term also encompasses "consisting of".

The recitation of numerical ranges by endpoints includes all numbers and fractions subsumed within that range, as well as the recited endpoints.

The term "about" as used herein when referring to a measurable value such as a parameter, an amount, a temporal duration, and the like, is meant to encompass variations of and from the specified value, in particular variations of +/-20% or less, preferably +/-10% or less, more preferably +/-5% or less, even more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

All documents cited in the present specification are hereby incorporated by reference in their entirety. In particular, the teachings of all documents herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, ensuing definitions are included to better appreciate the teaching of the present invention.

The term "nucleic acid" as used herein means a polymer of any length composed essentially of nucleotides, e.g., deoxyribonucleotides and/or ribonucleotides. Nucleic acids can comprise purine and/or pyrimidine bases and/or other natural (e.g., xanthine, inosine, hypoxanthine), chemically or biochemically modified (e.g., methylated), non-natural, or derivatised nucleotide bases. The backbone of nucleic acids can comprise sugars and phosphate groups, as can typically be found in RNA or DNA, and/or one or more modified or substituted sugars and/or one or more modified or substituted phosphate groups. The term "nucleic acid" further preferably encompasses DNA, RNA and DNA/RNA hybrid molecules, specifically including hnRNA, pre-mRNA, mRNA, cDNA, genomic DNA, amplification products, oligonucleotides, and synthetic (e.g. chemically synthesised) DNA, RNA or DNA/RNA hybrids. A "nucleic acid" can be double-stranded, partly double stranded, or single-stranded. Where single-stranded, the nucleic acid can be the sense strand or the antisense strand. In addition, nucleic acid can be circular or linear.

In a preferred embodiment, the nucleic acid comprising a promoter of the invention is DNA or RNA, more preferably DNA.

The term "recombinant nucleic acid" refers generally to a nucleic acid which is comprised of segments joined together using recombinant DNA technology. When a recombinant nucleic replicates in a host organism, the progeny nucleic acids are also encompassed within the term "recombinant nucleic acid".

Standard reference works setting forth the general principles of recombinant DNA technology include Molecular Cloning: A Laboratory Manual, 2nd ed., vol. 1-3, ed. Sambrook et al., Cold Spring Harbor Laboratory Press, Cold Spring Harbor, N.Y., 1989; Current Protocols in Molecular Biology, ed. Ausubel et al., Greene Publishing and Wiley-Interscience, New York, 1992 (with periodic updates) ("Ausubel *et al.* 1992"); Innis et al., PCR Protocols: A Guide to Methods and Applications, Academic Press: San Diego, 1990. General principles of microbiology are set forth, for example, in Davis, B. D. et al., Microbiology, 3rd edition, Harper & Row, publishers, Philadelphia, Pa. (1980).

By "promoter" is meant generally a region on a nucleic acid molecule, preferably DNA molecule, to which an RNA polymerase binds and initiates transcription. A promoter is preferably, but not necessarily, positioned upstream, i.e., 5', of the sequence the transcription of which it controls. The term encompasses both inducible and constitutive promoters, and may preferably encompass constitutive promoters.

The term "native promoter" refers to a promoter the nucleotide sequence of which is identical to that of a promoter present in nature, e.g., in a cell or organism in nature. The modifier "native promoter" thus relates to the sequence of the promoter and is not to be construed as requiring that the promoter be obtained or produced in any particular manner. By means of example and not limitation, the term would thus encompass promoters in their natural hosts, isolated there from, cloned and propagated using recombinant DNA technology, produced by an amplification method or generated by synthetic means, etc., insofar the sequence of such promoters would be the same or similar as of their counterparts occurring in nature.

A skilled person understands that the native sequence of the promoter of a given gene may differ between different species of *Lactobacillus* or *Streptococcus* and/or between different subspecies within a single species of *Lactobacillus* or *Streptococcus* and/or between different strains within a single species or subspecies of *Lactobacillus* or *Streptococcus,* due to natural genetic divergence between the said species, subspecies and/or strains. Thus, such divergent but found-in-nature promoter sequences would be considered native.

With use of the present invention, a skilled person is in general capable of predicting and identifying natural bacterial promoters, such as promoters of *Lactobacillus* or *Streptococcus.* Nevertheless, to offer added guidance, a natural promoter may be often identified by analysing a genomic sequence or part thereof from a bacterium, preferably from a *Lactobacillus* or *Streptococcus* species; identifying an open reading frame therein, i.e., a succession of coding nucleotide triplets starting with a translation initiation codon (preferably, ATG) and closing with a translation termination codon (e.g., TAA, TAG or TGA) and not containing any internal in-frame translation termination codon; and analysing the sequence upstream of the said translation initiation codon to locate the upstream-most translation initiation codon, upstream to which there occurs an in-frame translation termination codon. Preferably, the transcription of the so-identified open reading frame can be verified experimentally, such as, e.g., by Northern blotting or RT-PCR; and the transcription initiation site (e.g., adjacent to the upstream-most and/or, perhaps, one or more of the more downstream translation initiation codons) can be evaluated using, e.g., 5'-rapid amplification of cDNA ends method (5'-RACE).

Typically, the sequences 5' adjacent to and proximal to the upstream-most translation initiation codon (and/or, if experimental evidence so indicates, one or more of the more downstream translation initiation codons) may comprise the native promoter responsible for transcribing the said ORF. By means of a preferred example, when the first nucleotide of the translation initiation codon is denoted +1 (e.g., the A nucleotide of the ATG codon is +1) and the nucleotide directly 5' thereof is denoted -1, then the term "native promoter" may refer to the sequence from about -500 to about +50, e.g., from about -500 to about +20, from about -500 to about +10, from about -500 to about +5, from about -500 to about +2, or from about -500 to about -1; preferably from about -400 to about +50, e.g., in preferred examples, from about -400 to about +20, e.g., from about -400 to about +10, from about -400 to about +5, from about -400 to about +2 or from about -400 to about -1; more preferably from about -300 to about +50, e.g., in preferred examples, from about -300 to about +20, e.g., from about -300 to about +10, from about -300 to about +5, from about -300 to about +2 or from about -300 to about -1; such as, e.g., in preferred examples, from about -200 to about +50 and in further preferred examples, from about -200 to about +20, e.g., from about -200 to about +10, from about -200 to about +5, from about -200 to about +2 or from about -200 to about -1; or such as, e.g., in other preferred example from about -100 to about +50 and in further preferred examples from about -100 to about +20, e.g., from about -100 to about +10, from about -100 to about +5, from about -100 to about +2 or from about -100 to about -1; insofar the said sequence displays the promoter activity.

The use of functional variants of native *Lactobacillus* or *Streptococcus* promoters in recombinant nucleic acids of the invention is also contemplated. The term "variant" refers to a sequence which is substantially identical (*i.e.*, largely but not wholly identical) to a corresponding native sequence, e.g., to the sequence of a corresponding native *Lactobacillus* or *Streptococcus* promoter. "Substantially identical" refers to at least 60%, preferably at least 70% identical, more preferably at least 80% identical, e.g., at least 85% identical, even more preferably at least 90% identical, e.g., at least 91% identical, 92% identical, yet more preferably at least 93% identical, e.g., 94% identical, still more preferably at least 95% identical, e.g., at least 96% identical, even more preferably at least 97% identical, e.g., at least 98% identical, and most preferably at least 99% identical.

Preferably, a variant may display such degrees of identity to a recited nucleic acid when the whole sequence of the recited nucleic acid is queried in the sequence alignment (i.e., overall sequence identity). In an alternative, a variant may display such degrees of identity to a recited nucleic acid when a comparison window of at least about 50 consecutive nucleotides, more preferably at least about 100 consecutive nucleotides, even more preferably at least about 150 consecutive nucleotides and most preferably at least about 200 consecutive nucleotides, of the recited nucleic acid is queried in the sequence alignment (i.e., localised sequence identity). Sequence alignments and determination of sequence identity can be done, e.g., using the Basic Local Alignment Search Tool (BLAST) originally described by Altschul et al. 1990 (J Mol Biol 215: 403-10), such as the "Blast 2 sequences" algorithm described by Tatusova and Madden 1999 (FEMS Microbiol Lett 174: 247-250).

The use of functional fragments of native *Lactobacillus* or *Streptococcus* promoters in recombinant nucleic acids of the invention is also contemplated. As used herein, the term "fragment" refers to a sequence that has a 5' and/or 3' deletion of one or more nucleotides as compared to a native sequence, e.g., a native *Lactobacillus* or *Streptococcus* promoter or a variant thereof, but where the remaining nucleic acid sequence of the fragment is identical to the corresponding positions in the sequence of the native *Lactobacillus* or *Streptococcus* promoter or a variant thereof. The remaining sequence of a fragment can represents preferably at least 30%, e.g., at least 40%, more preferably at least 50%, e.g., at least 60%, even more preferably at least 70%, e.g., at least 80% or at least 85%, and still more preferably at least 90%, e.g., at least 95% or more of the nucleic acid sequence of the respective native *Lactobacillus* or *Streptococcus* promoter or variant thereof.

The term "functional" with reference to the variants and fragments of promoters as above refers to the fact that the particular variants and fragments will have at least partly retained the promoter activity, i.e., the capability to bind RNA polymerase and initiate transcription, of the corresponding native promoter. Preferably, such functional variants or functional fragments may retain at least 50% of the activity of the corresponding native promoter, e.g., at least 60%, more preferably at least 70%, e.g., at least 80%, yet more preferably at least 85%, e.g., at least 86%, at least 87%, at least 88% or 89%, still more preferably at least 90%, e.g., at least 91%, at least 92%, at least 93%, at least 94%, and most preferably at least 95%, e.g., at least 96%, at least 97%, and very preferably at least 98% or at least 99% of the activity of the corresponding native promoter. Also preferably, such functional variants or functional fragments may even have higher activity than the corresponding native promoter.

A skilled person can also appreciate that in embodiments the recombinant nucleic acids of the invention may even comprise more than one promoter and/or functional variant and/or functional fragment of the invention. For instance, said promoters, functional variants or functional fragments - which may be same or different - can be operably linked to and control the expression of distinct transcription units within the said recombinant nucleic acids. Alternatively or in addition, expression of a single transcription unit may be controlled by more than one promoter(s), functional variant(s) and/or functional fragment(s), linked operably thereto, which can be same or different. For example, operable association of more than one of the above elements having promoter activity with a single transcription unit may further increase the level of transcription of the said unit. By means of example and not limitation, such promoters, functional variants and/or functional fragments may be arranged sequentially, e.g., sequentially upstream of the respective transcription unit.

Yet alternatively, the invention also envisages recombinant nucleic acids comprising chimeric promoters including two or more portions derived from different promoters, functional variants and/or functional fragments of the invention, and together constituting a new promoter.

A skilled person is aware of techniques to evaluate the activity of promoters. For example, a nucleic acid sequence whose activity as a promoter is sought to be determined can be inserted into a recombinant reporter construct such that it is operably linked with a reporter sequence, preferably a reporter coding sequence, such as, e.g., green fluorescent protein (GFP) or chloramphenicol acetyl transferase (CAT), etc. and the expression and/or accumulation of the reporter mRNA (e.g., by Northern blotting, quantitative RT-PCR, etc.) and/or protein (e.g., by Western blotting, ELISA, measurement of fluorescence or enzymatic activity, etc.) is assayed when the said reporter construct is introduced into host cells or organisms of interest.

In an exemplary preferred embodiment, the expression can be measured for a protein heterologous to the organism in which the expression is measured, e.g., heterologous to bacteria, preferably heterologous to *Lactobacillus* or *Streptococcus,* even more preferably heterologous to *Lactobacillus rhamnosus* or *Streptococcus mutans.* For instance, in a preferred embodiment, expression in bacteria, preferably in *Lactobacillus* or *Streptococcus* may be assessed for a gene encoding a polypeptide of eukaryotic origin, even more preferably for any of the genes encoding the prophylactically and/or therapeutically relevant peptides, polypeptides or proteins intended for expression using the nucleic acids of the invention (as described elsewhere in this specification). Values so measured for the assayed nucleic acid sequences indicate the activity or strength of potential promoters comprised within such sequences. A skilled person also understands that to ensure the comparative nature of such promoter activity assays, conditions other than the assayed nucleic acid sequences should be kept about the same or, ideally, the same between the different assays. Such conditions may comprise, by means of example, the quantity of the reporter construct introduced into cells, the transformation method used the said introduction, the number and site of integration of such reporter constructs in the genome of the assayed recipient cells, and/or the state (e.g., growth phase, e.g., preferably exponential growth phase) of the recipient host cells at the time of the measurement, etc.

Accordingly, to realise functional variants or fragments of promoters, especially of promoters disclosed by the present invention, and with aid of the present disclosure, the skilled person would know to prepare such variants (e.g., by targeted or random mutagenesis) or fragments (e.g., by 5' and/or 3' truncation, e.g., by restriction digestion or PCR) and assay such variants or fragments for their promoter activity as above. Nevertheless, by means of further guidance and not limitation, it is noted that bacterial promoters often include consensus sequences adjacent to positions -10 and -35. Accordingly, functional fragments of bacterial, e.g., *Lactobacillus* or *Streptococcus* promoters, may preferably comprise at least sequences corresponding to positions in the native promoters from about -10 to about -35, more preferably from about -8 to about -40, even more preferably from about -5 to about -40 or from about -5 to about -45, and still more preferably from about -2 or -1 to about -50. Moreover, functional variants of bacterial, e.g., *Lactobacillus* or *Streptococcus* promoters, may preferably comprise consensus sequences adjacent to positions -10 and -35 as present in the native counterpart promoters or as known in the art.

An "operable linkage" is a linkage in which the regulatory DNA sequences and the DNA sequence sought to be expressed are connected in such a way as to permit expression.

For example, DNA sequences, such as, e.g., preferably a promoter and a heterologous open reading frame, are said to be operably linked if the nature of the linkage between the sequences does not (1) result in the introduction of a frame-shift mutation, (2) interfere with the ability of the promoter to direct the transcription of the open reading frame, or (3) interfere with the ability of the open reading frame to be transcribed by the promoter region sequence.

In an exemplary preferred embodiment, the said promoter may be positioned upstream of, *i.e.,* 5' of, the open reading frame(s) to which it is operably linked.

The precise nature of the regulatory regions needed for expression may vary from organism to organism, but shall in general include a promoter region which, in prokaryotes, contains both the promoter (which directs the initiation of RNA transcription) as well as the DNA sequences which, when transcribed into RNA, will signal the initiation of protein synthesis. Such regions will normally include those 5'-non-coding sequences involved with initiation of transcription and translation, such as the Pribnow-box (cf. TATA-box), Shine-Dalgarno sequence, and the like.

Advantageously, the respective translation initiation codon with which a given promoter of the invention is normally associated in nature may not be included in recombinant nucleic acids of the invention, such as to prevent translation initiation from such codons. For example, the 3' end of the promoter may be truncated at the -1 position or upstream thereof; alternatively, the translation initiation codon may be mutated (e.g., from ATG to a different codon); etc. Yet alternatively, the said native translation initiation codon may be present, and possibly also several (e.g., preferably ≤ 20, more preferably ≤ 10, yet more preferably ≤ 5, e.g., at most 1, 2, 3 or 4) subsequent codons of the open reading frame associated with the given promoter in nature, and a heterologous open reading frame of interest may be fused thereto in-frame to produce a fusion product.

The term "open reading frame" or ORF refers to a succession of coding nucleotide triplets starting with a translation initiation codon (preferably ATG) and closing with a translation termination codon (e.g., TAA, TAG or TGA) and not containing any internal in-frame translation termination codon, and potentially capable of encoding a polypeptide. Hence, the term may be synonymous with "coding sequence" as used in the art. In the recombinant nucleic acid of the invention, the translation initiation codons of the one or more ORFs may typically be associated with regulatory sequences controlling initiation of translation, e.g., with the Shine-Dalgarno sequence. It is also known that in bacteria, including *Lactobacillus* or *Streptococcus,* multi-cistronic units containing two or more sequential ORFs controlled by a common upstream promoter may be created by associating downstream translation initiation codons with the said sequences controlling such translation initiation.

The term "heterologous", when referring to the relationship between a given ORF and a promoter, means that the said promoter is not normally associated with, *i.e.*, is not normally controlling the transcription of, the said ORF in nature. In other words, the association is created by recombinant DNA techniques in the recombinant nucleic acids of the invention.

The term *"Lactobacillus"* or "*Lactobacillus* sp." generally refers to the genus *Lactobacillus* and encompasses any taxon (e.g., species, subspecies, strain) classified as belonging to such in the art. By means of example, *Lactobacillus* or *Lactobacillus* sp. includes the species *L. acetotolerans, L. acidifarinae, L. acidipiscis, L. acidophilus, L. agilis, L. algidus, L. alimentarius, L. amylolyticus, L. amylophilus, L. amylotrophicus, L. amylovorus, L. animalis, L. antri, L. apodemi, L. aviarius, L. bifermentans, L. brevis, L. buchneri, L. camelliae, L. casei, L. catenaformis, L. ceti, L. coleohominis, L. collinoides, L. composti, L. concavus, L. coryniformis, L. crispatus, L. crustorum, L. curvatus, L. delbrueckii, L. delbrueckii subsp. bulgaricus, L. delbrueckii subsp. lactis, L. diolivorans, L. equi, L. equigenerosi, L. farraginis, L. farciminis, L. fermentum, L. fornicalis, L. fructivorans, L. frumenti, L. fuchuensis, L. gallinarum, L. gasseri, L. gastricus, L. ghanensis, L. graminis, L. hammesii, L. hamsteri, L. harbinensis, L. hayakitensis, L. helveticus, L. hilgardii, L. homohiochii, L. iners, L. ingluviei, L. intestinalis, L. jensenii, L. johnsonii, L. kalixensis, L. kefiranofaciens, L. kefiri, L. kimchii, L. kitasatonis, L. kunkeei, L. leichmannii, L. lindneri, L. malefermentans, L. mali, L. manihotivorans, L. mindensis "L. mucosae, L. murinus, L. nagelii, L. namurensis, L. nantensis, L. oligofermentans, L. oris, L. panis, L. pantheris, L. parabrevis, L. parabuchneri, L. paracollinoides, L. parafarraginis, L. parakefiri, L. paralimentarius, L. paraplantarum, L. pentosus, L. perolens, L. plantarum, L. pontis, L. psittaci, L. rennini, L. reuteri, L. rhamnosus, L. rimae, L. rogosae, L. rossiae, L. ruminis, L. saerimneri, L. sakei, L. salivarius, L. sanfranciscensis, L. satsumensis, L. secaliphilus, L. sharpeae, L. siliginis, L. spicheri, L. suebicus, L. hailandensis, L. ultunensis, L. vaccinostercus, L. vaginalis, L. versmoldensis, L. vini, L. vitulinus, L. zeae, L. zymae* and any subspecies and strains thereof. Preferably the *Lactobacillus* may be *Lactobacillus rhamnosus, Lactobacillus casei, Lactobacillus plantarum* or *Lactobacillus salivarius,* even more preferably *L. rhamnosus* HN001.

The term *"Streptococcus"* or *"Streptococcus* sp." generally refers to the genus *Streptococcus* and encompasses any taxon (e.g., species, subspecies, strain) classified as belonging to such in the art. By means of example, *Streptococcus* or *Streptococcus* sp. includes the species *S. agalactiae, S. anginosus, S. bovis, S. canis, S. equi, S. iniae, S. mitis,* S. *mutans, S. oralis, S. parasanguinis, S. peroris, S. pneumoniae, S. pyogenes, S*. *ratti, S. salivarius, S. salivarius* ssp. *thermophilus, S. sanguinis, S. sobrinus, S. suis, S. uberis, S. vestibularis, S. viridans,* and any subspecies and strains thereof. Preferably the *Streptococcus* may be *Streptococcus mutans,* even more preferably *S*. *mutans* UA159. *Streptococcus mutans* is a lactic acid bacterium that normally colonizes dental surfaces, and as such an may be particularly suitable to serve as a host organism for delivery of molecules to the oral mucosa.

In preferred embodiments, the promoter is derived from *Lactobacillus* or *Streptococcus* as defined above, more preferably from the preferred *Lactobacillus* or *Streptococcus* taxons as defined above, esp. in the two preceding paragraphs.

In a related embodiment, the invention thus also provides a recombinant nucleic acid comprising a promoter operably linked to one or more open reading frames heterologous to the promoter, wherein the promoter is chosen from the group comprising or consisting of the native promoters of genes of *Lactobacillus* or *Streptococcus* listed under 1) to 25) in the Summary section, and functional variants and functional fragments of the said native promoters. It is also preferred that the promoter is chosen from the group comprising or consisting of nucleic acids set forth in Tables 1 and Table 2 below, and functional variants and functional fragments of the said native promoters. The promoter sequences set out in Tables 1 and 2 represent exemplary, but not limiting, native promoters associated with the expression of the genes listed under 1) to 25) above in *Lactobacillus rhamnosus* or *Streptococcus mutans,* respectively.

Said designations are clear to a skilled person and teach specific genes from various *Lactobacillus* or *Streptococcus* taxons (e.g., species, subspecies and strains), such as the preferred *Lactobacillus* or *Streptococcus* taxons described above. Nevertheless, by virtue of further guidance Table 1 below mentions unique Gene ID numbers which identify the said genes as isolated from *Lactobacillus rhamnosus* (particularly *L. rhamnosus* HN001), and Table 2 below mentions unique Gene ID numbers which identify the said genes as isolated form *Streptococcus mutans* (particularly *S. mutans* UA159). The Gene ID numbers uniquely identify the said genes in the "Entrez Gene" database of NCBI (www.ncbi.nlm.nih.gov/entrez/query.fcgi?db=gene) as described in Maglott et al. 2005. (Entrez Gene: gene-centered information at NCBI. Nucleic Acids Res. 33: D54-D58). The NCBI Reference Sequence accession numbers in said Tables provide particular nucleic acid sequence information.

Tables 1 and 2 further identify regions of the nucleic acids which may be considered as respective promoter regions.

**Table 1. Select genes and promoter regions identified in Lactobacillus rhamnosus (particularly L. rhamnosus HN001).**

| **GI-number** | **Gene / Protein name** | **Abbrev.** | **Locus** | **Gene location in NCBI Reference Sequence** | **Promoter region** |
|---|---|---|---|---|---|
| 199598845 | ribosimal protein S14* | rpsJ | LRH_03200 | NZ_ABWJ01000023.1:2589.25974 | 19083-19412 |
| 199588701 | nucleoid DNA-binding protein | *dnabp* | LRH_04048 | complement(NZ_ABWJ01000020.1/15543..15818) | c(15819-16051) |
| 199589325 | ribosomal protein S21 | *rpS21* | LRH_09358 | complement(NZ_ABWJ01000036.1:5953..6129) | c(6130-6401) |
| 199588341 | ribosomal protein S17* | rpsJ | LRH_03180 | NZ_ABWJ01000023.1/24203..24466 | 19083-19412 |
| 199597121 | 50S ribosomal protein L19 | rpIS | LRH_07296 | complement(NZ_ABWJ01000002.1/62543..62890) | c(62891-63094) |
| 199598859 | 50S ribosomal protein L17 | *map40* | LRH_03270 | NZ_ABWJ01000023.1:33024..33404 | 31990-32063 |
| 199598851 | ribosomal protein L15* | rpsJ | LRH_03230 | NZ_ABWJ01000023.1:28112..28552 | 19083-19412 |
| 199538872 | 50S ribosomal protein L13 | rplM | LRH_03335 | NZ_ABWJ01000023.1:42964..43410 | 42698-42963 |
| 199597445 | phosphoglycerate mutase 1 | *pgm1* | LRH_13389 | NZ_ABWJ01000004.1:98387..99076 | 98118-98386 |
| 199588838 | ribosomal protein S3* | *rpsJ* | LRH_03165 | NZ_ABWJ01000023.1:22898..23560 | 19083-19412 |
| 199597503 | ribosomal protein S4 | *rpS4* | LRH_06231 | NZ_ABWJ01000005.1:20167..20778 | 19992-20166 |
| 199597272 | glyceraldehyde-3-phosphate dehydrogenase | *cgpr* | LRH_05289 | NZ_ABWJ01000003.1:50087..51109 | 48208-49012 |

| | | | | | |
|---|---|---|---|---|---|
| *identical operon | | | | | |

**Table 2. Select genes and promoter regions identified in Streptococus mutans (particularly S. mutans UA159)**

| **GI- number** | **Gene / Protein name** | **Locus** | **Gene location in NCBI Reference Sequence NC_004350 (version 1)** | **Promoter region** |
|---|---|---|---|---|
| 1350920 | 30S ribosomal protein S10 | SMU.2026c | complement(1893020..1893130) | c1893131-1893647 |
| 24379304 | 50S ribosomal protein L27 | SMU.849 | 797718..798011 | 796862-797035 |
| 24378669 | 30S ribosomal protein S15 | SMU.154 | 156212..156481 | 155470-156212 |
| 24378669 | 30S ribosomal protein S16 | SMU.865 | 814687..814962 | 814285-814686 |
| 24379704 | 50S ribosomal protein L19 | SMU.1288 | complement(1214632..1214979) | c1214980-1215169 |
| 24380355 | 30S ribosomal protein S8 | SMU.2012 | complement(1887696..1888094) | c1888095-18883350 |
| 24380353 | 50S ribosomal protein L18* | SMU.2010 | complement(1886301..1886657) | c1886658-1886746 |
| 24378685 | 30S ribosomal protein S9 | SMU.170 | 170269..170661 | 169408-169768 |
| 24380352 | 30S ribosomal protein S5* | SMU.2009 | complement(1885788..1886282) | c1886658-1886746 |
| 15902260 | 50S ribosomal protein L17 | SMU.2000 | complement(1880033..1880419) | c1881377-1881421 |
| 24380345 | 30S ribosomal protein S13 | SMU.2003 | complement(1881823..1882188) | c1882569-1882686 |
| 24378855 | 30S ribosomal protein S7 | SMU.358 | 334526..3343996 | 334997-335164 |
| 24380350 | 50S ribosomal protein L15 | SMU.2007 | complement(1884859..1885299) | c1885300-1885591 |
| 24380465 | 30S ribosomal protein S4 | SMU.2135c | complement(1993236..1999847) | c1999848-1999950 |
| 24380354 | 50S ribosomal protein L6 | SMU.2011 | complement(1886747..1887283) | c1887284-1887695 |
| 161488819 | 30S ribosomal protein S3** | SMU.2021 | complement(1890900..1891553) | c1892786-1893019 |
| 24379074 | phosphoglyceromutase | SMU.74 | 74927..75637 | 74855-74926 |
| 24380367 | 50S ribosomal protein L3** | SMU.2025 | complement(1892159..1892785) | c1892786-1893019 |

| | | | | |
|---|---|---|---|---|
| *identical operon **identical operon | | | | |

On the basis hereof, a skilled person can identify and isolate homologues of the said genes from further taxons of *Lactobacillus* or *Streptococcus,* e.g., the preferred species, subspecies and strains as taught herein. "Homologues" as used herein refers to sequences, esp. genes, from two or more different taxons that are similar (e.g., preferably, may be substantially identical as defined herein) as a result of originating from a common ancestor. Homologues of the above genes from *Lactobacillus rhamnosus* or *Streptococcus mutans* preferably fulfil the same function in other *Lactobacillus* or *Streptococcus* taxons, respectively.

A skilled person can henceforth identify and isolate native promoters controlling the expression of genes listed in Tables 1 or 2 or homologues thereof from *Lactobacillus* or *Streptococcus* taxons (including promoters which control the expression of genes that are found in multi-cistronic transcription units and thus may not contain a promoter directly upstream of their translation initiation codon) following the teachings of this specification and using common knowledge in the art.

Accordingly, these promoters and homologues thereof (esp. from other *Lactobacillus* or *Streptococcus* taxons) and functional variants and functional derivatives thereof can be useful in recombinant nucleic acids of the invention for effecting expression of useful open reading frames in host cells, preferably in bacterial host cells, even more preferably in Gram-positive bacteria such as non-pathogenic and/or non-invasive Gram-positive bacteria, also preferably in lactic acid bacteria such as non-pathogenic and/or non-invasive lactic acid bacteria, still more preferably in such bacteria of the *Lactobacillus* sp. or *Streptococcus* sp., respectively.

The recombinant nucleic acids disclosed herein may further comprise a transcription terminator sequence 3' to the said one or more open reading frames. For instance, if only one open reading frame is present, the terminator sequence may be advantageously located downstream, i.e., 3', thereof. If the recombinant nucleic acid contains two or more ORFs, e.g., successively ordered and forming together a multi-cistronic transcription unit, the transcription terminator may be advantageously positioned 3' to the most downstream ORF.

The term "transcription terminator" refers to a sequence element at the end of a transcriptional unit which signals termination of transcription. Preferably, a transcription terminator for use in the present invention will signal termination of transcription in host cells intended for use with the recombinant nucleic acids of the invention, such as, e.g., in bacterial host cells, even more preferably in *Lactobacillus* or *Streptococcus.*

The recombinant nucleic acids as disclosed herein may further comprise an operator configured to control transcription from the promoter. As used herein, the term "operator" refers to a nucleotide sequence, preferably DNA sequence, which controls the initiation and/or maintenance of transcription of a sequence from a promoter.

Typically, an operator may be generally placed between a promoter and a downstream sequence the transcription of which the promoters controls. Usually, an operator is capable of binding a repressor polypeptide, whereby it reduces the transcription from the said promoter. A useful repressor can alternate between a state in which it binds the operator and a state in which it does not and such alternation may be advantageously controlled by an external condition, e.g., an external substance or a particular metabolite. Accordingly, in host cells comprising a compatible repressor, the inclusion of an operator in the nucleic acid of the invention may allow to control the activity of the promoter and expression therefrom. Exemplary operators - repressor systems include, e.g., the *lac* system (see, e.g., Nauta et al. 1996. Mol Microbiol 19: 1331-41), or the histidine biosynthesis system (see, e.g., Delorme et al. 1999. J Bacteriol 181: 2026-37). Operator sequences may be generally derived from bacterial chromosomes.

The recombinant nucleic acids as disclosed herein may further comprise sequences configured to effect insertion of the said recombinant nucleic acids into the genome, e.g., a chromosome, of a host cell.

For example, insertion of the nucleic acids of the invention into particular sites within a genome, e.g. chromosome, of a host cell may be facilitated by homologous recombination. For instance, the recombinant nucleic acids of the invention may comprise one or more regions of homology to the said site of integration within the genome e.g., a chromosome, of the host cell. The sequence at the said genome, e.g. chromosome, site may be natural, i.e., as occurring in nature, or may be an exogenous sequence introduced by previous genetic engineering.

For instance, the said region(s) of homology may be at least 50 bp, preferably at least 100 bp, e.g., at least 200 bp, more preferably at least 300 bp, e.g., at least 400 bp, even more preferably at least 500 bp, e.g., at least 600 bp or at least 700 bp, still more preferably at least 800 bp, e.g., at least 900 bp, or at least 1000 bp or more.

In a preferred example, two regions of homology may be included, one flanking each side of the expression unit(s) present in the nucleic acids of the invention. Such configuration may advantageously insert the relevant sequences, i.e., the ones effecting the expression of the open reading frames of interest from the promoters of the invention, in host cells. Ways of performing homologous recombination, especially in bacterial hosts, and selecting for recombinants, are generally known in the art. An exemplary and preferred method is, e.g., that of Steidler et al. 2003 (Nat Biotechnol 21: 785-789) and WO 2004/046346.

Hence, in a preferred embodiment, the invention also provides the recombinant nucleic acid when integrated into genome, e.g., a chromosome, preferably a bacterial chromosome, more preferably a *Lactobacillus* or *Streptococcus* chromosome. Such integration may be obtained in a host cell transformed with the said recombinant nucleic acid or a vector comprising such, as described elsewhere in this specification.

In a preferred embodiment, the one or more open reading frames linked to a promoter of the invention in the nucleic acids of the invention encode a peptide, polypeptide or protein.

As can be appreciated, the essence of the invention primarily concerns novel promoters and their uses and the nature of the said expression products, preferably peptides, polypeptides or proteins, is not to be limited in any way. Nevertheless, host cells such as bacterial host cells have been reported as means for *in vitro* production and/or *in vivo* delivery of relevant expression products of viral, prokaryotic or eukaryotic origin, including peptides, polypeptides or proteins useful in prevention or therapy of disease in man and animals.

Accordingly, in an embodiment, the said one or more open reading frames of the recombinant nucleic acids disclosed herein may encode an expression product, preferably a peptide, polypeptide or protein, capable of eliciting a prophylactic and/or therapeutic response in a subject, preferably in a human or animal subject. Without limitation, said one or more open reading frames of the recombinant nucleic acids disclosed herein may encode an antigen and/or a non-vaccinogenic prophylactically and/or therapeutically active peptide, polypeptide or protein.

As used herein, the term "antigen" generally refers to a substance that evokes an immune response, including humoral immunity and/or cellular immunity response, and that is capable of binding with a product, e.g., an antibody or a T cell, of the immune response. Hence, in a preferred example, an antigen requires a functioning immune system of a subject to which it is administered to elicit a physiological response from such a subject. The "antigen" as intended herein also encompasses "self-antigens" which do not provoke an immune response in a healthy individual but would do so in a person suffering from auto-immune disease, i.e. the failure of an organism to recognize its own constituent parts (down to the sub-molecular levels) as "self", which results in an immune response against its own cells and tissues. Any disease that results from such an aberrant immune response is termed an autoimmune disease. Accordingly, the "antigen" as intended herein also encompasses a (physiologically active) protein which would not provoke an immune response in a healthy individual but would do so in a person genetically deficient in said protein. In addition, the "antigen" as intended herein also encompasses an allergen which would not provoke an immune response in a healthy individual but would do so in a person suffering from an allergic disease.

An antigen as intended herein may be derived from any polypeptide to which an immune response in a human or animal subject would be therapeutically useful, e.g., from a pathogen, e.g., from a viral, prokaryotic (e.g., bacterial) or eukaryotic pathogen, from a non-physiological protein (e.g., a protein derived from cancer tissue), from allergen (e.g., for eliciting immune tolerance), etc. An antigen could also be a metabolite of a protein. As an example, the antigen could be a polysaccharide, a lipid or other. Strong promoters as described here could drive the expression of the necessary enzymes to synthesize or assemble said polysaccharide, lipid or other.

The term "a non-vaccinogenic prophylactically and/or therapeutically active peptide, polypeptide or protein" refers generally to a peptide, polypeptide or protein that, in a human or animal subject to which it is administered, does not elicit an immune response against itself and is able to achieve a prophylactic and/or therapeutic effect. Hence, the prophylactic and/or therapeutic effect of such a peptide, polypeptide or protein would be expected to be directly linked to its own natural biological function whereby it can achieve particular effects in a body of a subject; rather than producing a prophylactic and/or therapeutic effect by acting as an immunogenic and/or immunoprotective antigen in the subject. Hence, the non-vaccinogenic prophylactically and/or therapeutically active peptide, polypeptide or protein should be biologically active in its expressed form or, at least, must be converted into the biologically active form once released from the expressing host cell. Preferably, such biologically active form of the said peptide, polypeptide or protein may display a secondary and preferably also tertiary conformation which is the same or closely analogous to its native configuration.

Preferably, the non-vaccinogenic prophylactically and/or therapeutically active peptide, polypeptide or protein is also non-toxic and non-pathogenic.

In a preferred embodiment, the non-vaccinogenic prophylactically and/or therapeutically active peptide, polypeptide or protein may be derived from human or animal, and may preferably correspond to the same taxon as the human or animal subject to which it is to be administered.

Non-limiting examples of suitable non-vaccinogenic prophylactically and/or therapeutically active peptides, polypeptides or proteins include ones which are capable of functioning locally or systemically, e.g., is/are capable of exerting endocrine activities affecting local or whole-body metabolism and/or is/are capable of the regulation of the activities of cells belonging to the immunohaemopoeitic system and/or is/are capable of affecting the viability, growth and differentiation of a variety of normal or neoplastic cells in the body or affecting the immune regulation or induction of acute phase inflammatory responses to injury and infection and/or is/are capable of enhancing or inducing resistance to infection of cells and tissues mediated by chemokines acting on their target cell receptors, or the proliferation of epithelial cells or the promotion of wound healing and/or is/are capable of modulating the expression or production of substances by cells in the body.

Specific examples of such peptides, polypeptides and proteins include, without limitation, insulin, growth hormone, prolactin, calcitonin, luteinising hormone, parathyroid hormone, somatostatin, thyroid stimulating hormone, vasoactive intestinal polypeptide, cytokines such as IL-2, IL-3, IL-4, IL-5, IL-6, IL-7, IL-9, IL-10, IL-11, IL-12, IL-13, any of IL-14 to IL-32, GM-CSF, M-CSF, SCF, IFNs, EPO,G-CSF, LIF, OSM, CNTF, GH, PRL, the TNF family of cytokines, e.g., TNFα, TNFβ, CD40, CD27 or FAS ligands, the IL-1 family of cytokines, the fibroblast growth factor family, the platelet derived growth factors, transforming growth factors and nerve growth factors, the epidermal growth factor family of cytokines, the insulin related cytokines, etc. Alternatively, the therapeutically active polypeptide can be a receptor or antagonist for the therapeutically active polypeptides as defined above. Alternatively, the therapeutically active polypeptide can be a neutralizing antibody, or the likes thereof. Further specific examples of such suitable polypeptides are listed, e.g., in WO 96/11277, page 14, lines 1-30, incorporated herein by reference; in WO 97/14806, page 12, line 1 through page 13, line 27, incorporated herein by reference; or US 5,559,007, col. 8, line 31 through col. 9, line 9, incorporated by reference herein. In an example, said non-vaccinogenic prophylactically and/or therapeutically active peptide, polypeptide or protein may be IL-10, more preferably hIL-10, glucagon-like peptide-1 (GLP-1), more preferably hGLP-1, glucagon-like peptide-2 (GLP-2), more preferably hGLP-2, trefoil factors (TFF, e.g., TFF1, 2 and/or 3), or PYY, more preferably hPYY.

Accordingly, in an embodiment the recombinant nucleic acid disclosed herein encodes an antigen and/or a non-vaccinogenic prophylactically and/or therapeutically active peptide, polypeptide or protein, wherein the said antigen is capable of eliciting an immune response, preferably protective immune response or immune tolerance response, in a human or animal subject, and/or the said non-vaccinogenic prophylactically and/or therapeutically active peptide, polypeptide or protein is capable of producing a prophylactic and/or therapeutic effect in a human or animal subject.

WO 97/14806 further specifically discloses co-expression of antigens with immune response stimulatory molecules, such as, e.g., interleukins, e.g., IL-2 or IL-6, by bacteria. Accordingly, such co-expression using the promoters of the invention is also contemplated.

The open reading frame as disclosed herein may further comprise a sequence encoding a secretion signal in phase with a polypeptide encoded by the ORF. This advantageously allows for secretion of the expressed polypeptide from the host cell and thereby may facilitate, e.g., isolation or delivery.

Typically, a secretion signal sequence represents an about 16 to about 35 amino acid segment, usually containing hydrophobic amino acids that become embedded in the lipid bilayer membrane, and thereby allow for the secretion of an accompanying protein or peptide sequence from the host cell, and which usually is cleaved from that protein or peptide. Preferably, the secretion signal sequence may be so-active in a host cell intended for use with the nucleic acid comprising the said signal sequence, e.g., a bacterial host cell, preferably a lactic acid bacterium, more preferably *Lactobacillus* or *Streptococcus.*

Secretion signal sequences active in suitable host cells are known in the art; exemplary signal sequences include those of usp45 such as usp45N4 (see, e.g., WO 2008/084115) and others. Preferably, the signal sequence is located between the promoter sequence and the ORF, *i.e.* the signal sequence is located 3' from the promoter sequence and precedes the ORF of the polypeptide of interest.

Further disclosed is a vector comprising the recombinant nucleic acid of the invention.

As used herein, "vector" refers to a nucleic acid molecule, typically DNA, to which nucleic acid fragments may be inserted and cloned, i.e., propagated. Hence, a vector will typically contain one or more unique restriction sites, and may be capable of autonomous replication in a defined host or vehicle organism such that the cloned sequence is reproducible. Vectors may include, without limitation, plasmids, phagemids, bacteriophages, bacteriophage-derived vectors, PAC, BAC, linear nucleic acids, e.g., linear DNA, etc., as appropriate (see, e.g., Sambrook *et al.,* 1989; Ausubel 1992).

Factors of importance in selecting a particular vector, e.g., a plasmid, include *inter alia:* the ease with which recipient cells that contain the vector may be recognized and selected from those recipient cells which do not contain the vector; the number of copies of the vector which are desired in a particular host; and whether it is desirable to be able to "shuttle" the vector between host cells of different species. Preferred prokaryotic vectors include plasmids such as those capable of replication in *E. coli* (such as, for example, pBR322, ColE1, pSC101, pUC19, etc.). Such plasmids are described in, e.g., Sambrook *et al.,* 1989; Ausubel 1992. Particularly preferred vectors may be those able to replicate in *E. coli* (or other Gram negative bacteria) as well as in another host cell of interest, such as in a Gram positive bacterium, a lactic acid bacterium, preferably *Lactobacillus* or *Streptococcus.* Other preferred vectors may be those able to replicate and/or shuttle between one or more Gram positive bacteria but not in Gram negative bacteria.

Further disclosed is a host cell transformed with the recombinant nucleic acid and/or with the vector as taught herein. For example, such transformation may be useful for propagation and maintenance of the said nucleic acid or vectors.

Alternatively or in addition, and advantageously, a transformed host cell will be capable of transcribing the open reading frame(s) of the nucleic acid of the invention using the promoters of the invention and, preferably, expressing the expression products, preferably one or more polypeptides, encoded by the said open reading frames.

Hence, disclosed is also the use of the recombinant nucleic acid or vector of the invention for achieving expression of expression products, preferably one or more polypeptides, encoded by the said open reading frames, in a host cell. Preferably, the expression products, e.g., polypeptides, may be heterologous, *i.e.* exogenous, to the said host cell.

Preferably, a host cell will be a prokaryotic cell, for example a bacterial cell, more preferably a non-pathogenic and/or non-invasive bacterium, yet more preferably a Gram-positive bacterium, still more preferably a lactic acid bacterium (e.g., *Lactococcus* spp., *Streptococcus* spp., *Lactobacillus* spp., *Leuconostoc* spp., *Pediococcus* spp., *Brevibacterium* spp., *Propionibacterium* spp. or *Bifidobacterium* spp.), very preferably a *Lactobacillus* or *Streptococcus* bacterium and most preferably a *Lactobacillus rhamnosus* or *Streptococcus mutans* bacterium, e.g., as defined above; insofar the promoters of the invention are suitably active in the said host cells.

The recombinant nucleic acid or the vector as taught herein may be present in the host cell extra-chromosomally, preferably autonomously replicating using an own origin of replication, or may be integrated into bacterial genomic DNA, e.g., bacterial chromosome, e.g., *Lactobacillus* or *Streptococcus* chromosome. In the latter case, a single or multiple copies of the said nucleic acid may be integrated, preferably a single copy; the integration may occur at a random site of the chromosome or, as described above, at a predetermined site thereof, preferably at a predetermined site, such as, in a preferred non-limiting example, in the *thyA* locus of *Lactobacillus* or *Streptococcus,* for instance as described by Steidler *et al.* (2003, *supra)* or WO 2004/046346, which is specifically incorporated by reference herein.

Further disclosed is a host cell as defined herein, wherein the said one or more open reading frames encode a peptide, polypeptide or protein capable of eliciting a prophylactic and/or therapeutic response or immunogenic response in a subject, preferably in a human or animal subject, preferably the said one or more open reading frames encode an antigen and/or a non-vaccinogenic prophylactically and/or therapeutically active peptide, polypeptide or protein, even more preferably the said antigen is capable of eliciting an immune response, preferably an immune tolerance response, in a human or animal subject, and/or the said non-vaccinogenic prophylactically and/or therapeutically active peptide, polypeptide or protein is capable of producing a prophylactic and/or therapeutic effect in a human or animal subject.

Also disclosed is a method for recombinant expression of an expression product, preferably a polypeptide, of interest comprising:
a) culturing the host cell comprising a recombinant nucleic acid or vector as taught herein, wherein the said one or more open reading frames encode the expression product, preferably polypeptide, of interest, and
b) isolating the expression product, preferably polypeptide, of interest produced by the host cell in the said culturing.

A skilled person generally knows and can further optimise culturing conditions, e.g., temperature, presence and concentration of necessary nutrients, oxygenation, stirring, inoculation, etc. that allow for expression of the polypeptides in host cells, preferably host cells as taught herein.

A skilled person also knows and can further optimise purification techniques for the isolation of the expression products, such as, preferably peptides, polypeptides or proteins, expressed by the said host cells. For example, suitable techniques of protein isolation may involve lysis of the host organism (e.g., when the product accumulates intracellularly) by, e.g., mechanical or enzymatic disruption of cell wall, and removal of cellular debris, or supernatant collection (e.g., when the product is secreted), removal of non-protein components, such as DNA and lipopolysaccharides, ammonium sulphate precipitation, size exclusion chromatography, e.g., to enrich for the desired protein, affinity chromatography, etc.

Further disclosed herein is the use of the host cell transformed with the recombinant nucleic acid and/or the vector as taught herein for the manufacture of a medicament for delivery of expression product(s), preferably peptide(s), polypeptide(s) or protein(s), encoded by the one or more open reading frames comprised within the said recombinant nucleic acid to a human or animal.

In a related aspect, the invention provides a method for delivery of a polypeptide encoded by the one or more open reading frames comprised within the recombinant nucleic acid as taught herein to human or animal in need thereof, comprising administering to the said human or animal a therapeutically effective amount of host cells transformed with the said nucleic acid and/or vector as taught herein. The animal may preferably be a mammal, such as, e.g., domestic animals, farm animals, zoo animals, sport animals, pet and experimental animals such as dogs, cats, guinea pigs, rabbits, rats, mice, horses, cattle, cows; primates such as apes, monkeys, orang-utans, and chimpanzees; canids such as dogs and wolves; felids such as cats, lions, and tigers; equids such as horses, donkeys, and zebras; food animals such as cows, pigs, and sheep; ungulates such as deer and giraffes; rodents such as mice, rats, hamsters and guinea pigs; and so on. Generally, the term "subject" or "patient" may be used interchangeably and particularly refer to animals, preferably warm-blooded animals, more preferably vertebrates, even more preferably mammals, still more preferably primates, and specifically includes human patients and non-human animals, mammals and primates. Preferred patients may be human subjects.

As used herein, the terms "treat" or "treatment" refer to both therapeutic treatment and prophylactic or preventative measures, wherein the object is to prevent or slow down (lessen) an undesired physiological change or disorder. A "human or animal in need of treatment" includes ones that would benefit from treatment of a given condition.

The term "therapeutically effective amount" refers to an amount of a therapeutic substance or composition effective to treat a disease or disorder in a subject, e.g., human or animal, i.e., to obtain a desired local or systemic effect and performance. By means of example, a therapeutically effective amount of bacteria may comprise at least 1 bacterium, or at least 10 bacteria, or at least 10² bacteria, or at least 10³ bacteria, or at least 10⁴ bacteria, or at least 10⁵ bacteria, or at least 10⁶ bacteria, or at least 10⁷ bacteria, or at least 10⁸ bacteria, or at least 10⁹, or at least 10¹⁰, or at least 10¹¹, or at least 10¹², or at least 10¹³, or at least 10¹⁴, or at least 10¹⁵, or more host cells, e.g., bacteria, e.g., in a single or repeated dose.

The host cells as taught herein may be administered alone or in combination with one or more active compounds. The latter can be administered before, after or simultaneously with the administration of the said host cells.

A number of prior art disclosures on the delivery of antigens and/or prophylactically and/or therapeutically active peptides, polypeptides or proteins exist, and it shall be appreciated that such disclosures may be further advantageously modified with the strong promoters of the present disclosure. By means of example and not limitation, bacterial delivery of interleukins in particular IL-10 for treating colitis (e.g., see WO 00/23471), delivery of antigens as vaccines (e.g., WO 97/14806), delivery of GLP-2 and related analogs may be used to treat short bowel disease, Crohn's disease, osteoporosis and as adjuvant therapy during cancer chemotherapy, etc. Furthermore, bacterial delivery of trefoil peptides may be used to treat diseases of the alimentary canal (see, e.g., WO 01/02570). In particular, the use of trefoil proteins or peptides for treatment of disorders of and damage to the alimentary canal, including the mouth, oesophagus, stomach, and large and small intestine, as well as for the protection and treatment of tissues that lie outside the alimentary canal are described in WO 97/38712 and WO 92/14837. These proteins can be used either to treat lesions in these areas or to inhibit the formation of lesions. These lesions can be caused by: radiation therapy or chemotherapy for the treatment of cancer, any other drug including alcohol which damages the alimentary canal, accidental exposure to radiation or to a caustic substance, infection, a digestive disorder including but not limited to non-ulcer dyspepsia, gastritis, peptic or duodenal ulcer, gastric cancer, MALT lymphoma, Menetier's syndrome, gastro-oesophageal reflux disease, Crohn's disease, ulcerative colitis and acute colitis of chemical, bacterial or obscure origin. Trefoil peptides are particularly useful to treat acute colitis. Further therapeutic applications are envisioned using the promoters and host cells of the invention.

Further non-limiting examples of the types of diseases treatable in humans or animals by delivery of prophylactic and/or therapeutic peptides, polypeptides or proteins as taught herein include, but are not limited to, e.g., inflammatory bowel diseases including Crohn's disease and ulcerative colitis (treatable with, e.g., IL-Ira or IL-10 or trefoil peptides); autoimmune diseases, including but not limited to psoriasis, rheumatoid arthritis, lupus erythematosus (treatable with, e.g., IL-Ira or IL-10 or the relevant auto-antigen); allergic diseases including but not limited to asthma, food allergies, (treatable with the relevant allergen); celiac disease (treatable with gluten allergens); neurological disorders including, but not limited to Alzheimer's disease, Parkinson's disease and amyotrophic lateral sclerosis (treatable with, e.g., brain derived neurotropic factor and ciliary neurotropic factor); cancer (treatable with, e.g., IL-1, colony stimulating factors or interferon-W); osteoporosis (treatable with, e.g., transforming growth factor f3); diabetes (treatable with, e.g., insulin); cardiovascular disease (treatable with, e.g., tissue plasminogen activator); atherosclerosis (treatable with, e.g., cytokines and cytokine antagonists); hemophilia (treatable with, e.g., clotting factors); degenerative liver disease (treatable with, e.g., hepatocyte growth factor or interferon a); pulmonary diseases such as cystic fibrosis (treatable with, e.g., alpha antitrypsin); obesity; pathogen infections, e.g., viral or bacterial infections (treatable with any number of the above-mentioned compositions or antigens); etc.

The skilled reader shall appreciate that the herein specifically recited diseases are only exemplary and their recitation is in no way intended to confine the use of the reagents disclosed herein, e.g., the promoters, nucleic acids, vectors and host cells as taught herein, to these particular diseases. Instead, a skilled reader understands that the reagents disclosed herein can be used to express in principle any expression products, preferably polypeptides, of interest, which may be of therapeutic relevance in not only the recited ones but also in various further diseases or conditions of humans and animals. Consequently, once a suitable expression product, preferably a polypeptide, e.g., an antigen and/or a non-vaccinogenic prophylactically and/or therapeutically active peptide, polypeptide or protein, has been chosen or determined for a given ailment, a skilled person would be able to achieve its expression, isolation and/or delivery using the present reagents.

Further disclosed is treatment of diseases in other animals including dogs, horses, cats and birds. Diseases in dogs include but are not limited to canine distemper (paramyxovirus), canine hepatitis (adenovirus Cav-1), kennel cough or laryngotracheitis (adenovirus Cav-2), infectious canine enteritis (coronavirus) and haemorrhagic enteritis (parvovirus).

Diseases in cats include but are not limited to viral rhinotracheitis (herpesvirus), feline caliciviral disease (calicivirus), feline infectious peritonitis (parvovirus) and feline leukaemia (feline leukaemia virus). Other viral diseases in horses and birds are also contemplated as being treatable using the methods and compositions of the invention. To this purpose, the use of microorganisms expressing recombinant interferons will be particularly preferred.

A further aspect relates to a pharmaceutical composition comprising the host cell transformed with the nucleic acid and/or the vector as taught herein.

Preferably, such formulation comprise a therapeutically effective amount of the host cells as disclosed herein and a pharmaceutically acceptable carrier, i.e., one or more pharmaceutically acceptable carrier substances and/or additives, e.g., buffers, carriers, excipients, stabilisers, etc.

The term "pharmaceutically acceptable" as used herein is consistent with the art and means compatible with the other ingredients of a pharmaceutical composition and not deleterious to the recipient thereof.

The recombinant host cells as taught herein can be suspended in a pharmaceutical formulation for administration to the human or animal having the disease to be treated. Such pharmaceutical formulations include but are not limited to live host cells and a medium suitable for administration. The recombinant host cells may be lyophilized in the presence of common excipients such as lactose, other sugars, alkaline and/or alkali earth stearate, carbonate and/or sulphate (for example, magnesium stearate, sodium carbonate and sodium sulphate), kaolin, silica, flavorants and aromas.

Host cells so-lyophilized may be prepared in the form of capsules, tablets, granulates and powders, each of which may be administered by the oral route.

Alternatively, some recombinant bacteria may be prepared as aqueous suspensions in suitable media, or lyophilized bacteria may be suspended in a suitable medium just prior to use, such medium including the excipients referred to herein and other excipients such as glucose, glycine and sodium saccharinate.

For oral administration, gastroresistant oral dosage forms may be formulated, which dosage forms may also include compounds providing controlled release of the host cells and thereby provide controlled release of the desired protein encoded therein. For example, the oral dosage form (including tablets, pellets, granulates, powders) may be coated with a thin layer of excipient (usually polymers, cellulosic derivatives and/or lipophilic materials) that resists dissolution or disruption in the stomach, but not in the intestine, thereby allowing transit through the stomach in favour of disintegration, dissolution and absorption in the intestine.

The oral dosage form may be designed to allow slow release of the host cells and of the recombinant protein thereof, for instance as controlled release, sustained release, prolonged release, sustained action tablets or capsules. These dosage forms usually contain conventional and well known excipients, such as lipophilic, polymeric, cellulosic, insoluble, swellable excipients. Controlled release formulations may also be used for any other delivery sites including intestinal, colon, bioadhesion or sublingual delivery (i.e., dental mucosal delivery) and bronchial delivery. When the compositions as taught herein are to be administered rectally or vaginally, pharmaceutical formulations may include suppositories and creams. In this instance, the host cells are suspended in a mixture of common excipients also including lipids. Each of the aforementioned formulations are well known in the art and are described, for example, in the following references: Hansel et al. 1990 ("Pharmaceutical dosage forms and drug delivery systems" 5th edition, William and Wilkins), Chien 1992 ("Novel drug delivery system" 2nd edition, M. Dekker), Prescott et al. 1989 ("Novel drug delivery", J. Wiley & Sons) and Cazzaniga et al. 1994 ("Oral delayed release system for colonic specific delivery" Int. J. Pharm).

Preferably, an enema formulation may be used for rectal administration. The term "enema" is used to cover liquid preparations intended for rectal use. The enema may be usually supplied in single-dose containers and contains one or more active substances dissolved or dispersed in water, glycerol or macrogols or other suitable solvents.

Hence, according with the present disclosure, recombinant host cells encoding a desired gene may be preferably administered to the animal or human via mucosal, e.g., an oral, nasal, rectal, vaginal or bronchial route by any one of the state-of-the art formulations applicable to the specific route. Dosages of host cells for administration will vary depending upon any number of factors including the type of bacteria and the gene encoded thereby, the type and severity of the disease to be treated and the route of administration to be used.

Thus, precise dosages cannot be defined for each and every embodiment of the invention, but will be readily apparent to those skilled in the art once armed with the present invention. The dosage could be anyhow determined on a case by case way by measuring the serum level concentrations of the recombinant protein after administration of predetermined numbers of cells, using well known methods, such as those known as ELISA or Biacore (see examples). The analysis of the kinetic profile and half life of the delivered recombinant protein provides sufficient information to allow the determination of an effective dosage range for the transformed host cells.

In an embodiment, when the host cells express an antigen, the disclosure may thus also provide a vaccine.

The term "vaccine" identifies a pharmaceutically acceptable composition that, when administered in an effective amount to an animal or human subject, is capable of inducing antibodies to an immunogen comprised in the vaccine and/or elicits protective immunity in the subject.

The vaccine of the invention would comprise the host cells transformed with the nucleic acids or vectors as taught herein and further optionally an excipient. Such vaccines may also comprise an adjuvant, i.e., a compound or composition that enhances the immune response to an antigen. Adjuvants include, but are not limited to, complete Freund's adjuvant, incomplete Freund's adjuvant, saponin, mineral gels such as aluminum hydroxide, surface active substances such as lysolecithin, pluronic polyols, polyanions, peptides, oil or hydrocarbon emulsions, and potentially useful pharmaceutically acceptable human adjuvants such as BCG (bacille Calmetle-Guerin) and *Corynebacterium parvum.*

In further preferred embodiments the present invention relates to the use of a host cell as defined herein for delivering (or for the manufacture of a medicament for delivery of) a peptide, polypeptide or protein encoded by the said one or more open reading frames of the recombinant nucleic acid to a human or animal, preferably said product is an antigen and/or a non-vaccinogenic prophylactically and/or therapeutically active peptide, polypeptide or protein as defined above.

Further disclosed is a pharmaceutical composition comprising the host cell as defined in herein. Also disclosed is a host cell as defined in herein, for use as a medicament, or for use in treating a disease or condition in which the administration of said one or more (preferably heterologous) expression products is capable of eliciting a prophylactic and/or therapeutic effect.

The invention is further illustrated with examples that are not to be considered limiting.

### EXAMPLES

### Example 1: Isolation of strong promoters from Lactobacillus rhamnosus and Streptococcus mutans

Highly expressed proteins were identified by abundant protein spots in a 1 D gel.

The excised protein samples were reduced and alkylated with iodoacetamide, i.e. carbamidomethylated, and digested with trypsin that cleaves after lysine and arginine residues. The resulting peptides were concentrated on a ZipTip micropurification column and eluted onto an anchorchip target for analysis on a Bruker Autoflex III MALDI TOF/TOF instrument. The peptide mixture was analyzed in positive reflector mode for accurate peptide mass determination and 5-10 of the peptides selected for analysis by MS/MS fragmentation for partial peptide sequencing. The MS and MS/MS spectra were combined and used for a database search in an in-house protein database using the Mascot software. Matching proteins are found in the database based on the number of matching peptide masses and the peptide fragment masses. The protein identification is based on a probability-scoring algorithm (www.matrixscience.com) and the significant best matching protein were recorded.

The identified database protein sequences are shown in Table 1 and 2 above.

### Example 2 Promoter activity

The promoters of the genes shown in Tables 1 and 2 above are isolated by PCR, and cloned upstream of and in operable linkage to a secretable hIL-10 gene.

The resulting constructs are introduced, on an extrachromosomal vector or on a vector which undergoes chromosomal integration, into *Lactobacillus rhamnosus* or *Streptococcus mutans,* respectively. The bacteria are cultured in a suitable rich medium at conditions conducive to protein expression by the bacteria.

Expression level of hIL-10 is quantified by measuring the protein in the supernatant using ELISA and the hIL-10 proteins are visualized by analyzing the equivalent of 1 ml culture by western blot.

The expression level determined indicates above average to strong expression driven by the promoters of genes listed in Tables 1 and 2 in the respective bacteria, for heterologous expression.

In view of the foregoing, it will be appreciated that the invention described herein inter alia relates to the following items:
1. A recombinant nucleic acid comprising a promoter, being a native promoter from a *Lactobacillus* species or a functional variant or functional fragment thereof, operably linked to one or more open reading frames heterologous to the promoter, wherein the promoter is chosen from the group comprising or consisting of the native promoters of genes of *Lactobacillus,* preferably of *Lactobacillus rhamnosus,* for: 1) ribosomal protein S14 and ribosomal protein S17 and ribosomal protein L15 and ribosomal protein S3 *(rpsJ),* 2) nucleoid DNA-binding protein (*dnabp*), 3) ribosomal protein S21 *(rpS21),* 4) 50S ribosomal protein L19 (*rplS*), 5) 50S ribosomal protein L17 (*rnap40*), 6) 50S ribosomal protein L13 (*rplM*), *7)* phosphoglycerate mutase 1 (*pgm1*), 8) ribosomal protein S4 *(rpS4),* 9) glyceraldehyde-3-phosphate dehydrogenase *(cggr),* and functional variants and functional fragments of the said native promoters.
2. A recombinant nucleic acid comprising a promoter, being a native promoter from a *Streptococcus* species or a functional variant or functional fragment thereof, operably linked to one or more open reading frames heterologous to the promoter, wherein the promoter is chosen from the group comprising or consisting of the native promoters of genes of *Streptococcus,* preferably of *Streptococcus mutans,* for: 10) 30S ribosomal protein S10, 11) 50S ribosomal protein L27, 12) 30S ribosomal protein S15, 13) 30S ribosomal protein S16, 14) 50S ribosomal protein L19, 15) 30S ribosomal protein S8, 16) 50S ribosomal protein L18 and 30S ribosomal protein S5, 17) 30S ribosomal protein S9, 18) 50S ribosomal protein L17, 19) 30S ribosomal protein S13, 20) 30S ribosomal protein S7, 21) 50S ribosomal protein L15, 22) 30S ribosomal protein S4, 23) 50S ribosomal protein L6, 24) 30S ribosomal protein S3 and 50S ribosomal protein L3, 25) phosphoglyceromutase, and functional variants and functional fragments of the said native promoters.
3. The recombinant nucleic acid according to item 1, wherein the promoter is chosen from the group comprising or consisting of nucleic acids as set forth in Table 1, and homologues thereof, and functional variants and functional fragments thereof.
4. The recombinant nucleic acid according to item 2, wherein the promoter is chosen from the group comprising or consisting of nucleic acids as set forth in Table 2, and homologues thereof, and functional variants and functional fragments thereof.
5. The recombinant nucleic acid according to any one of items 1 to 4, further comprising an operator configured to control transcription from the said promoter.
6. The recombinant nucleic acid according to any one of items 1 to 5, further comprising sequences configured to effect insertion of the said recombinant nucleic acid into the chromosome of a host cell, preferably by homologous recombination.
7. The recombinant nucleic acid according to any one of items 1 to 6, further comprising a secretion signal sequence.
8. The recombinant nucleic acid according to any of items 1 to 7, wherein the said one or more open reading frames encode a peptide, polypeptide or protein capable of eliciting a prophylactic, therapeutic or immunogenic response in a subject, preferably in a human or animal subject.
9. The recombinant nucleic acid according to item 8, wherein the said one or more open reading frames encode an antigen and/or a non-vaccinogenic prophylactically and/or therapeutically active peptide, polypeptide or protein.
10. The recombinant nucleic acid according to item 9, wherein the said antigen is capable of eliciting an immune response, preferably an immune tolerance response, in a human or animal subject, and/or the said non-vaccinogenic prophylactically and/or therapeutically active peptide, polypeptide or protein is capable of producing a prophylactic and/or therapeutic effect in a human or animal subject.
11. The recombinant nucleic acid according to item 9, wherein the said antigen is capable of eliciting an immune response and used as a vaccine in a human or animal subject.
12. A vector comprising the recombinant nucleic acid as defined in any of items 1 to 11.
13. A host cell comprising the recombinant nucleic acid as defined in any of items 1 to 11 or with the vector of item 12.
14. The host cell according to item 13, wherein said promoter is present in the chromosome of said host cell, and wherein said promoter is operably linked to one or more open reading frames heterologous to said promoter.
15. The host cell according to any of items 13 or 14, which is a non-pathogenic and non-invasive bacterium, preferably Gram-positive bacterium, more preferably lactic acid bacterium, even more preferably *Lactobacillus* or *Streptococcus* bacterium.
16. Use of the recombinant nucleic acid of any of items 1 to 11 or the vector of item 12 for achieving expression of expression products encoded by the said one or more open reading frames, in a host cell.
17. A method for recombinant expression of a peptide, polypeptide or protein of interest comprising:
   a) culturing the host cell as defined in any of items 13 to 15, wherein the said one or more open reading frames encode the peptide, polypeptide or protein of interest,
   b) isolating the peptide, polypeptide or protein of interest produced by the host cell in the said culturing.
18. Use of the host cell as defined in any of items 13 to 15 for the manufacture of a medicament for delivery of a peptide, polypeptide or protein encoded by the said one or more open reading frames of the recombinant nucleic acid to a human or animal.
19. A pharmaceutical composition comprising the host cell as defined in any of items 13 to 15.
20. A host cell according to any of items 13 to 15 for use as a medicament.

## Claims

1. A recombinant nucleic acid comprising a promoter, operably linked to one or more open reading frames heterologous to the promoter, wherein the promoter is chosen from the group consisting of the native promoters of genes of *Streptococcus,* for:
a) 30S ribosomal protein S10,
b) 50S ribosomal protein L27,
c) 30S ribosomal protein S15,
d) 30S ribosomal protein S16,
e) 50S ribosomal protein L19,
f) 30S ribosomal protein S8,
g) 50S ribosomal protein L18 and 30S ribosomal protein S5,
h) 30S ribosomal protein S9,
i) 50S ribosomal protein L17,
j) 30S ribosomal protein S13,
k) 30S ribosomal protein S7,
l) 50S ribosomal protein L15,
m) 30S ribosomal protein S4,
n) 50S ribosomal protein L6,
o) 30S ribosomal protein S3 and 50S ribosomal protein L3,
p) phosphoglyceromutase, and
q) functional variants and functional fragments thereof having an overall sequence identity of at least 80%, at least 90%, or at least 95% thereto.

2. A recombinant nucleic acid comprising a promoter, operably linked to one or more open reading frames heterologous to the promoter, wherein the promoter is chosen from the group consisting of the native promoters of genes of *Lactobacillus,* for:
a) ribosomal protein S14 and ribosomal protein S17 and ribosomal protein L15 and ribosomal protein S3 (*rpsJ*),
b) nucleoid DNA-binding protein (*dnabp*),
c) ribosomal protein S21 (*rpS21*),
d) 50S ribosomal protein L19 (*rplS*),
e) 50S ribosomal protein L17 (*rnap40*),
f) 50S ribosomal protein L13 (*rplM*),
g) phosphoglycerate mutase 1 (*pgm1*),
h) ribosomal protein S4 (*rpS4*)*,* and
i) functional variants and functional fragments thereof having an overall sequence identity of at least 80%, at least 90%, or at least 95% thereto.

3. The recombinant nucleic acid according to claim 1, wherein the promoter is chosen from the group consisting of the native promoters of genes of *Streptococcus mutans,* and homologues thereof, and functional variants and functional fragments thereof having an overall sequence identity of at least 80%, at least 90%, or at least 95% thereto.

4. The recombinant nucleic acid according to claim 3, wherein the promoter is chosen from the group consisting of the native promoters of genes of *Streptococcus mutans* UA159, and homologues thereof, and functional variants and functional fragments thereof having an overall sequence identity of at least 80%, at least 90%, or at least 95% thereto.

5. The recombinant nucleic acid according to claim 2, wherein the promoter is chosen from the group consisting of the native promoters of genes of *Lactobacillus rhamnosus,* and homologues thereof, and functional variants and functional fragments thereof having an overall sequence identity of at least 80%, at least 90%, or at least 95% thereto.

6. The recombinant nucleic acid according to claim 5, wherein the promoter is chosen from the group consisting of the native promoters of genes of *Lactobacillus rhamnosus* HN001, and homologues thereof, and functional variants and functional fragments thereof having an overall sequence identity of at least 80%, at least 90%, or at least 95% thereto.

7. The recombinant nucleic acid according to any one of claims 1 to 6, wherein the functional variants and functional fragments of the native promoters of genes of *Streptococcus* or *Lactobacillus* retain at least 80%, at least 90%, or at least 95% of the native promoter activity.

8. The recombinant nucleic acid according to any one of claims 1 to 7, further comprising an operator configured to control transcription from the said promoter.

9. The recombinant nucleic acid according to any one of claims 1 to 8, further comprising sequences configured to effect insertion of the said recombinant nucleic acid into the chromosome of a host cell, preferably by homologous recombination.

10. The recombinant nucleic acid according to any one of claims 1 to 9, further comprising a secretion signal sequence.

11. The recombinant nucleic acid according to any one of claims 1 to 10, wherein the said one or more open reading frames encode a peptide, polypeptide or protein capable of eliciting a prophylactic, therapeutic or immunogenic response in a subject, preferably in a human or animal subject.

12. The recombinant nucleic acid according to claim 11, wherein the said one or more open reading frames encode an antigen and/or a non-vaccinogenic prophylactically and/or therapeutically active peptide, polypeptide or protein.

13. The recombinant nucleic acid according to claim 12, wherein the said non-vaccinogenic prophylactically and/or a non-vaccinogenic prophylactically and/or therapeutically active peptide, polypeptide or protein is selected from: insulin, a growth hormone, prolactin, calcitonin, luteinising hormone, parathyroid hormone, somatostatin, thyroid stimulating hormone, vasoactive intestinal polypeptide, a cytokine, a receptor or antagonist thereof, a neutralizing antibody, IL-10, GLP-1, GLP-2, trefoil factors and PYY.

14. The recombinant nucleic acid according to claim 13, wherein the cytokine is selected from: IL-2, IL-3, IL-4, IL-5, IL-6, IL-7, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31 and IL-32, GM-CSF, M-CSF, SCF, IFNs, EPO, G-CSF, LIF, OSM, CNTF, GH, PRL, a TNF family member, an IL-1 family member, a fibroblast growth factor family member, a platelet-derived growth factor, a transforming growth factor, a nerve growth factor, an epidermal growth factor family member, and an insulin related cytokine.

15. The recombinant nucleic acid according to any one of claims 12 to 14, wherein the said antigen is capable of eliciting an immune response, preferably an immune tolerance response, in a human or animal subject, and/or the said non-vaccinogenic prophylactically and/or therapeutically active peptide, polypeptide or protein is capable of producing a prophylactic and/or therapeutic effect in a human or animal subject.

16. The recombinant nucleic acid according to any one of claims 12 to 15, wherein the said antigen is capable of eliciting an immune response and is used as a vaccine in a human or animal subject.

17. A vector comprising the recombinant nucleic acid according to any one of claims 1 to 16.

18. A host cell comprising the recombinant nucleic acid according to any one of claims 1 to 16 or the vector according to claim 17.

19. The host cell according to claim 18, wherein said promoter is present in the chromosome of said host cell, and wherein said promoter is operably linked to one or more open reading frames heterologous to said promoter.

20. The host cell according to claim 18 or claim 19, which is a non-pathogenic and non-invasive bacterium, preferably a Gram-positive bacterium.

21. The host cell according to claim 20, which is a lactic acid bacterium, preferably a *Streptococcus* or a *Lactobacillus* bacterium.

22. The host cell according to claim 21, which is *Streptococcus mutans* or *Lactobacillus rhamnosus.*

23. Use of the recombinant nucleic acid according to any one of claims 1 to 16, or the vector according to claim 17, for achieving expression of expression products encoded by the said one or more open reading frames, in a host cell.

24. A method for recombinant expression of a peptide, polypeptide or protein of interest comprising:
a) culturing the host cell according to any one of claims 18 to 22, wherein the said one or more open reading frames encode the peptide, polypeptide or protein of interest,
b) isolating the peptide, polypeptide or protein of interest produced by the host cell in the said culturing.

25. The host cell according to any one of claims 18 to 22, for use in the delivery of the peptide, polypeptide or protein encoded by the said one or more open reading frames of the recombinant nucleic acid, to a human or animal.

26. A pharmaceutical composition comprising the host cell according to any one of claims 18 to 22.

27. The pharmaceutical composition according to claim 26, prepared in the form of capsules, tablets, granulates, powders, or as an aqueous suspension.

28. A host cell according to any one of claims 18 to 22 for use as a medicament.

29. A host cell according to any one of claims 18 to 22 for use as a vaccine, wherein the host cell expresses an antigen.

30. A host cell according to any one of claims 18 to 22 for use in treating a disease or condition selected from: an inflammatory bowel disease, an autoimmune disease, an allergic disease, celiac disease, a neurological disorder, cancer, osteoporosis, diabetes, a cardiovascular disease, atherosclerosis, hemophilia, degenerative liver disease, a pulmonary disease, obesity or a pathogen infection.

31. The host cell for the use according to claim 30, wherein the inflammatory bowel disease is Crohn's disease or ulcerative colitis; the autoimmune disease is psoriasis, rheumatoid arthritis or lupus erythematosus; the allergic disease is asthma or food allergies; the neurological disorder is Alzheimer's disease, Parkinson's disease or amyotrophic lateral sclerosis; the pulmonary disease is cystic fibrosis; or the pathogen infection is a viral or bacterial infection.

32. The host cell for the use according to claim 30 or claim 31, wherein:
a) the disease or condition is inflammatory bowel disease, and the therapeutic peptide, polypeptide or protein is selected from: IL-Ira, IL-10 and trefoil peptides;
b) the disease or condition is an autoimmune disease, and the therapeutic peptide, polypeptide or protein is selected from: IL-Ira or IL-10 and the relevant auto-antigen;
c) the disease or condition is an allergic disease and the therapeutic peptide, polypeptide or protein is the relevant allergen;
d) the disease or condition is celiac disease, and the therapeutic peptide, polypeptide or protein is selected from gluten allergens;
e) the disease or condition is a neurological disorder, and the therapeutic peptide, polypeptide or protein is selected from: brain derived neurotropic factor and ciliary neurotropic factor;
f) the disease or condition is cancer, and the therapeutic peptide, polypeptide or protein is selected from: IL-1, colony stimulating factors and interferon-W;
g) the disease or condition is osteoporosis, and the therapeutic peptide, polypeptide or protein is transforming growth factor f3;
h) the disease or condition is diabetes, and the therapeutic peptide, polypeptide or protein is insulin;
i) the disease or condition is cardiovascular disease, and the therapeutic peptide, polypeptide or protein is tissue plasminogen activator;
j) the disease or condition is atherosclerosis, and the therapeutic peptide, polypeptide or protein is selected from: cytokines and cytokine antagonists;
k) the disease or condition is hemophilia, and the therapeutic peptide, polypeptide or protein is selected from clotting factors;
l) the disease or condition is degenerative liver disease, and the therapeutic peptide, polypeptide or protein is selected from: hepatocyte growth factor and interferon a; or
m) the disease or condition is pulmonary disease, and the therapeutic peptide, polypeptide or protein is alpha antitrypsin.
